Europäisches Patentamt

European Patent Office

Office européen des brevets

(19)

(11) Numéro de publication: **0 237 384**
**A1**

(12) **DEMANDE DE BREVET EUROPEEN**

(21) Numéro de dépôt: 87400282.7

(22) Date de dépôt: 06.02.87

(51) Int. Cl.⁴: **C 07 K 15/00**
C 12 P 21/00, C 12 N 5/00,
C 12 N 15/00, G 01 N 33/569,
G 01 N 33/577
//(C12P21/00,C12R1:91)

(30) Priorité: 06.02.86 FR 8601620

(43) Date de publication de la demande:
16.09.87 Bulletin 87/38

(84) Etats contractants désignés:
AT BE CH DE ES FR GB GR IT LI LU NL SE

(71) Demandeur: CHEMUNEX
41 rue Barrault
F-75013 Paris (FR)

(72) Inventeur: **Strosberg, Arthur Donny**
**66 rue de Javel**
**F-75015 Paris (FR)**

**Goavec, Martine**
**150 rue de Tolbiac**
**F-75013 Paris (FR)**

**Bringer, Christine**
**3 rue du Pont Couilly**
**F-77174 Villeneuve-le-comte (FR)**

**Fuhrmann, Benoit**
**7 bis rue Bausset**
**F-75015 Paris (FR)**

(74) Mandataire: **Nony, Michel et al**
**Cabinet NONY & CIE 29, rue Cambacérès**
**F-75008 Paris (FR)**

Le (Les) microorganisme(s) a (ont) été déposé(s) auprès l'Institut Pasteur (CNCM) sous le(s) numéro(s) I - 500; I - 501; I - 509.

(54) **Nouveaux anticorps anti-levures capables de reconnaître plusieurs levures ou moisissures, lignées cellulaires hybrides produisant de tels anticorps, leur préparation et leur application à la détection de levures ou de moisissures.**

(57) Anticorps polyspécifiques, caractérisés par le fait qu'ils sont capables de reconnaître au moins une levure et au moins une moisissure, et en particulier, anticorps polyspécifiques ne reconnaissant pas les ferments lactiques ; lignées cellulaires hybrides sécrétant de tels anticorps ; procédé de préparation de ces anticorps et leur application à la détection de levures et/ou de moisissures, notamment dans les produits alimentaires et en particulier dans les produits lactés.

EP 0 237 384 A1

**0 237 384**

## Description

Nouveaux anticorps anti-levures capables de reconnaître plusieurs levures ou moisissures, lignées cellulaires hybrides produisant de tels anticorps, leur préparation et leur application à la détection de levures ou de moisissures.

La présente invention a pour objet des nouveaux anticorps capables de reconnaître plusieurs levures et moisissures, des lignées cellulaires hybrides produisant de tels anticorps, la préparation desdits anticorps, et leur application à la détection et éventuellement à la numération de levures ou de moisissures.

Il est usuel dans l'industrie agro-alimentaire, de réaliser la détection de microorganismes dans les produits alimentaires par des techniques microbiologiques classiques. Certes, la culture des microorganismes constitue la meilleure méthode de détection ; cependant une telle méthode a pour inconvénient d'être longue puisqu'elle nécessite de 1 à plusieurs jours pour donner des résultats utilisables et, de plus, elle ne peut être mise en oeuvre que dans des laboratoires spécialisés dans la culture des microorganismes.

Il s'est donc avéré nécessaire de pouvoir disposer de méthodes très sensibles et plus rapides, pouvant être mises en oeuvre sans nécessiter l'intervention de laboratoires spécialisés. C'est ainsi qu'à été proposée l'application des méthodes immunoenzymatiques, mises au point pour le diagnostic des maladies de l'homme et des maladies de l'animal, à la détection de microorganismes toxiques dans les produits alimentaires.

La présente invention a pour but de permettre la détection et éventuellement la quantification de levures et/ou de moisissures présentes dans les substances les plus diverses, aussi bien dans le cas où les levures ou moisissures sont des constituants de ces substances, que dans le cas où elles en sont des contaminants.

Les levures et les moisissures sont des champignons, qui selon la taxonomie font partie de la division des Eumycota dans laquelle on dénombre cinq sous-divisions ; les Mastigomycotina, les Zygomycotina, les Ascomycotina, les Basidiomycotina et les Deuteromycotina (Fungi Imperfecti) (Clasification des champignons selon AINSWORTH in Smith's Introduction to Industrial Mycology, A.H.S. ONIONS et al, 1981).

On compte environ 400 levures qui comprennent notamment les genres suivants : Candida, Saccharomyces, Trichosporon, Debaryomyces, Torulopsis, Cryptococcus, Brettanomyces, Hansenula, Kluyveromyces, Kloeckera, Nadsonia, Pichia, Rhodotorula, Zygosaccharomyces (PROCESS BIOCHEMISTRY, November 1970) "Immunological methods in industry" I. CAMPBELL p.48).

Les moisissures comprennent notamment les genres suivants : Penicillium, Aspergillus, Mucor, Geotrichum, Alternaria, Ulocladium, Trichoderma, Phialophora, Fusarium, Eurotium.

La reproduction des levures et des moisissures est de type végétatif avec formation d'un mycelium, et/ou de type sexué.

Les levures et les moisissures sont des microorganismes de grande taille (supérieure à 2μm), délimitées par une paroi riche en polysaccharide (80% à 90%).

(V. FARKAS Biosynthesis of Cell Wall of Fungi, Microbiological Reviews, June 1979 p.117-144).

Un produit alimentaire peut être contaminé par une ou plusieurs levures ou moisissures ; il faudrait donc pouvoir disposer, pour réaliser le test de détection immunoenzymatiques, non seulement d'anticorps spécifiques du contaminant qui a servi à les produire, mais également, d'anticorps dirigés contre d'autres levures et/ou d'autres moisissures.

La présente invention a donc pour objet d'obtenir des anticorps, aussi bien polyclonaux que monoclonaux, qui répondent à un besoin important, qui est celui de la détection de la présence de levures et/ou de moisissures, et notamment de levures ou moisissures contaminantes toxiques, dans différentes substances, en particulier dans des produits alimentaires, qui permettent de détecter la présence dans ces derniers de plusieurs levures et/ou de moisissures, et qui permettent la réalisation d'un test immunologique sensible et rapide, ne nécessitant pas l'intervention de spécialistes et, par suite, apte à être mis sous la forme d'une trousse de détection aisément utilisable par un personnel sans formation.

La présente invention a pour objet des anticorps polyspécifiques, caractérisés par le fait qu'ils sont capables de reconnaître au moins une levure et au moins une moisissure.

L'invention concerne aussi de tels anticorps, qui sont capables de reconnaître en outre au moins une seconde levure.

La présente invention a notamment pour objet des anticorps anti-levure capables de reconnaître non seulement la levure utilisée pour les préparer, mais également d'autres levures et/ou moisissures.

L'expression "anticorps-anti-levure" désigne des anticorps qui ont été obtenus par immunisation d'un animal à l'aide d'une levure.

L'invention concerne notamment des anticorps, tels que définis ci-dessus, qui sont capables de reconnaître au moins une première et une deuxième levures, différentes par leur genre ou leur espèce, prises dans le groupe constitué par les levures des genres Candida, Saccharomyces, Trichosporon, Debaryomyces, Torulopsis, Hansenula, Rhodotorula, Pichia, Cryptococcus, Kluyveromyces et Zygosaccharomyces.

Les anticorps polyspécifiques de l'invention sont en particulier ceux qui sont capables de reconnaître une première levure de même espèce que la levure utilisée pour préparer lesdits anticorps, prise dans le groupe de levures qui comprend Debaryomyces hansenii, Candida pseudotropicalis, Candida tropicalis, Candida albicans, Torulopsis glabrata, Trichosporon cutaneum, Saccharomyces cerevisiae, Saccharomyces carlsbergensis, Cryptococcus neoformans, et au moins une autre levure, différente par son genre ou son espèce, de la levure utilisée pour les préparer, et prise notamment dans le groupe des levures qui comprend Debaryomyces hansenii, Candida pseudotropicalis, Candida tropicalis, Candida albicans, Torulopsis glabrata, Trichosporon cutaneum, Saccharomyces cerevisiae, Saccharomyces carlsbergensis, Cryptococcus neoformans.

Les anticorps de l'invention sont notamment ceux qui sont capables de reconnaître, outre au moins une levure, au moins une moisissure (sous forme de mycelium ou de spore) choisie dans le groupe constitué par les moisissures des genres Penicillium, Aspergillus, Mucor, Geotrichum, Alternaria, Ulocladium, Trichoderma, Phialophora, Fusarium et Eurotium.

Parmi les anticorps de l'invention, on citera plus particulièrement les anticorps, tels que définis ci-dessus, qui ne donnent pas de réactivité croisée, ou qui ne donnent qu'une faible réactivité croisée, par exemple au plus égale à 10%, avec au moins un ferment lactique, ce qui permet de les utiliser pour la détection de levures et/ou de moisissures contaminantes dans les produits alimentaires obtenus par fermentation lactique. Parmi ces anticorps, on mentionnera notamment ceux qui ne donnent pas de réaction croisée avec au moins un ferment lactique choisi parmi Streptococcus thermophilus, Lactobacillus bulgaricus, Lactobacillus helveticus, Lactobacillus acidophilus, Lactobacillus brevis, Lactobacillus lactis, Bifidobacterium bifidum, Streptococcus lactis, Streptococcus diacetylactis, Lactobacillus casei casei et Lactobacillus casei rhamnosus.

Selon un mode de réalisation avantageux de l'invention, les anticorps polyspécifiques sont constitués par une composition comprenant en combinaison au moins deux préparations d'anticorps polyspécifiques tels que définis plus haut, dans le but d'étendre le domaine de détection de ladite composition d'anticorps.

Les anticorps polyspécifiques de l'invention peuvent être des anticorps polyclonaux ou monoclonaux.

Parmi les anticorps monoclonaux, on citera en particulier ceux qui sont obtenus à partir des lignées cellulaires hybrides décrites ci-après et déposées sous les numéros I-500, I-501, I-509.

Les anticorps ou leurs fragments peuvent en outre être modifiés par marquage et/ou fixés sur un support solide.

La présente invention a également pour objet un procédé de préparation d'anticorps polyspécifique tels que définis ci-dessus, caractérisé par le fait qu'il comprend une première étape, dans laquelle on immunise des animaux vertébrés, selon les techniques connues, et une seconde étape dans laquelle on recueille les anticorps des animaux immunisés.

Les animaux utilisés pour l'immunisation sont par exemple le lapin, la chèvre ou la souris.

L'immunisation est effectuée de préférence par des injections répétées de cellules de levures, et en particulier de cellules des genres mentionnés précédemment. On peut effectuer par exemple l'immunisation à l'aide de levures choisies dans le groupe constitué par Debaryomyces hansenii, Candida pseudotropicalis, Candida tropicalis, Candida albicans, Torulopsis glabrata, Trichosporon cutaneum, Saccharomyces cerevisiae, Saccharomyces carlsbergensis et Cryptococcus neoformans.

Selon un mode d'exécution préféré du procédé conforme à l'invention, les cellules de levures utilisées au cours de l'étape d'immunisation sont des cellules préalablement tuées par traitement thermique et/ou chimique.

Selon un autre mode d'exécution, pour la première injection auxdits animaux, les cellules de levures sont mises en suspension dans l'adjuvant complet de Freund ou en solution physiologique, et la deuxième injection et les injections éventuelles suivantes sont réalisées avec des cellules de levure en suspension dans de l'adjuvant incomplet de Freund ou en solution physiologique.

La seconde étape du procédé consiste à prélever du sang de l'animal immunisé et à en séparer le sérum qui contient les anticorps polyclonaux recherchés.

Les anticorps sériques sont ensuite isolés selon les techniques de fractionnement connues. Le sérum séparé au cours de la seconde étape est traité par exemple par chromatographie d'affinité pour recueillir la fraction d'anticorps spécifiques du sérum, dont l'utilisation permet d'augmenter la sensibilité de la détection des levures dans les milieux soumis aux tests de détection.

Selon un mode de mise en oeuvre particulier du procédé de l'invention, au cours d'une troisième étape, les anticorps polyspécifiques sont soumis à un traitement propre à rendre visible la réaction ultérieure anticorps-antigènes de détection de levures ou moisissures dans un milieu à tester, ce traitement consistant à les marquer avec un agent traceur et/ou à les fixer sur un support solide.

Les anticorps polyspécifiques obtenus peuvent être soumis à un traitement d'immunoabsorption par des levures susceptibles de donner lieu à une réaction croisée, pour obtenir des anticorps monospécifiques qui reconnaissent une seule levure. Les anticorps monospécifiques ainsi obtenus peuvent être éventuellement fragmentés, marqués, et/ou fixés sur un support solide.

Les anticorps de l'invention peuvent être des immunoglobines G ou M.

Dans les cas où l'on préfère obtenir les anticorps sous la forme d'immunoglobulines G (IgG), le procédé d'immunisation est réalisé de façon à provoquer une réponse immunitaire secondaire, selon les méthodes connues. On peut par exemple injecter l'immunogène une fois par semaine pendant trois semaines, puis faire un rappel au bout de deux ou trois mois. On administre l'immunogène par exemple par injection intradermique, sous-cutanée ou intraveineuse.

Pour sélectionner les anticorps qui sont capables de reconnaître au moins deux levures, on fait réagir la préparation des anticorps selon les techniques immunologiques connues basées sur la réaction antigène-anticorps, avec au moins une levure, autre que celle ayant servi à l'immunisation, que l'on souhaite pouvoir détecter à l'aide de l'anticorps préparé. On sélectionne de façon analogue les anticorps capables de reconnaître en outre au moins une moisissure.

Selon un autre mode d'exécution du procédé de l'invention, la seconde étape comprend un premier stade

3

qui consiste à fusionner des cellules lymphoïdes des animaux immunisés avec des cellules de vertébrés cultivables in vitro (cellules immortalisées) et un deuxième stade qui consiste à cloner les cellules hybrides obtenues, selon les techniques connues en soi. On sélectionne ensuite les clones sécrétant les anticorps ayant les propriétés désirées, c'est-à-dire ceux qui répondent aux critères mentionnés précédemment.

Au cours d'un troisième stade de la seconde étape, les clones peuvent être injectés à des animaux de la même espèce que ceux utilisés pour l'immunisation, sensibilisés par un agent approprié pour former des ascites qui produisent des quantités importantes des anticorps monoclonaux recherchés.

Les cellules mères utilisées dans l'opération de fusion cellulaire sont par exemple des splénocytes que l'on recueille selon les techniques usuelles.

On effectue l'étape de fusion des lymphocytes prélevés, avec des cellules provenant de lignées monoclonales cultivables in vitro, par exemple des cellules de myélome. On choisit de préférence des cellules de myélome non sécrétantes.

La fusion des cellules est une technique bien connue qui consiste essentiellement à mélanger des lymphocytes de l'animal immunisé et les cellules immortalisées provenant de lignées monoclonales, en présence d'un agent promoteur de fusion qui peut être, par exemple, le polyéthylèneglycol. On opère par exemple par la technique de KOHLER et MILSTEIN, NATURE, 256, 495-497 (1975).

Après incubation dans des conditions convenables, les cellules sont lavées et mises en culture sur un milieu sélectif permettant la croissance des cellules hybrides uniquement. Il est connu par exemple que les cellules de myélome, qui sont dépourvues de l'enzyme hypoxanthine-guanine-phosphoribosyl-transférase, ne se reproduisent pas sur les milieux contenant de l'hypoxanthine, de l'aminoptérine et de la thymidine. Dans ces conditions, les cellules n'ayant pas fusionné ne peuvent pas se reproduire sur le milieu sélectif utilisé.

Les cellules de myélome utilisées dans le procédé de l'invention sont par exemple des cellules de myélome de souris PIE gracieusement fournies par F. GIDOSSI DISA (Institut J. Monod, Paris). Ces cellules non sécrétantes ont été sélectionnées pour leur sensibilité à l'aminoptérine et cultivées sur un milieu approprié contenant des substances nutritives. Un tel milieu peut être constitué par exemple d'un milieu RPMI 1640 défini par Moore et al. (Culture of Normal Human Leucocytes, J.A.M.A. 199, 519-524, 1967) contenant 10% de sérum de veau foetal, 2mM de glutamine, 1mM de pyruvate de sodium, 100 UI/ml de pénicilline et 100μg/ml de streptomycine ; ce milieu sera dénommé ci-après milieu RPMI complet.

On sélectionne ensuite, après un temps de culture suffisant (par exemple 15 jours après la fusion) les surnageants de culture contenant des anticorps répondant aux critères mentionnés ci-dessus. Pour mettre en oeuvre cette sélection, on utilise les méthodes immunologiques classiques fondées sur la réaction antigène-anticorps. On utilise par exemple un test immuno-enzymatique. On effectue ensuite le clonage des cellules retenues à chaque étape des différents critères de sélection.

Le clonage est avantageusement effectué dans des microplaques et les puits desdites microplaques contenant des clones uniques sont ensuite sélectionnés et leur surnageants sont testés, par exemple à l'aide d'un test ELISA, pour ne retenir dans un premier temps que les clones sécrétant des anticorps anti-levure qui ont une haute affinité pour la levure utilisée pour l'immunisation, et qui sont stables.

L'invention s'étend aux lignées cellulaires hybrides sécrétant des anticorps répondant aux critères énoncés précédemment.

La présente invention a également pour objet les lignées cellulaires de cellules hybrides déposées sous les numéros I-500, I-501 et I-509 décrites ci-après.

Les lignées cellulaires I-500, I-501 et I-509 ont été obtenues à partir de cultures d'hybridomes résultant d'une fusion somatique entre splénocytes de souris Balb/c et cellules de myélome vecteur PIE non sécrétantes (respectivement DG3 et IG7) dans un milieu de culture constitué par un milieu RPMI 1640 additionné de L-glutamine, de streptomycine, de pénicilline, de pyruvate de sodium et de sérum de feau foetal, pH 7,2, à une température de 37°C, le repiquage étant effectué dès que la culture atteint $0,8.10^6$ cellules/ml dans le milieu du culture.

Les anticorps obtenus selon le procédé conforme à la présente invention peuvent être conservés par exemple par congélation à -20°C. Après de fréquentes décongélations et congélations on n'a pas observé de diminution notable de leur titre.

Les anticorps de l'invention peuvent être marqués ou encore être fixés sur un support solide permettant leur utilisation comme agents immunoadsorbants dans des méthodes de chromatographie d'affinité ou comme réactifs dans les méthodes d'analyse fondées sur la réaction antigène-anticorps (techniques radioimmunologiques, techniques d'immunofluorescence, techniques immunoenzymatiques, notamment).

Ledit support solide peut être réalisé en tout matériau solide, biologique ou synthétique, doué de propriétés adsorbantes ou capable de fixer un agent de couplage.

Ces matériaux sont connus et décrits dans la littérature. Parmi les matériaux solides capables de fixer les anticorps par adsorption, on citera par exemple le polystyrène, le polypropylène, les latex, etc... Parmi les matériaux solides utilisables pour fixer les anticorps par covalence à l'aide d'un agent de couplage, on peut citer notamment le dextran, la cellulose, leurs dérivés aminés (diéthylaminoéthylcellulose ou diéthylaminoéthyldextran), etc...

Le support solide peut se présenter par exemple sous forme de disques, de tubes, de baguettes, de billes, ou de plaques de microtitration.

Les agents de couplage permettant de fixer par covalence les anticorps sur le support solide sont des dérivés bifonctionnels tels que des dialdéhydes, des quinones, etc...

Les anticorps peuvent également être fixés sur des supports solides minéraux, par exemple selon les méthodes décrites dans les brevets français 2.319.176, 2.403.556 et 2.403.098.

Les anticorps selon l'invention conviennent particulièrement bien pour la détection de levures ou de moisissures, par exemple des levures ou des moisissures susceptibles de contaminer les produits alimentaires, notamment par les techniques d'immunofluorescence, les techniques immunoenzymatiques, en particulier la méthode ELISA, et les techniques radioimmunologiques, directes ou indirectes.

A cet effet, les anticorps conformes à l'invention peuvent être modifiés par marquage, notamment avec un traceur radioactif (tel que l'iode ou l'indium, par exemple), fluorescent (par exemple isothiocyanate de fluoresceine ou de rhodamine), chromogène, enzymatique (par exemple peroxydase ou phosphatase alcaline) ou colloïdal (or par exemple).

L'activité enzymatique éventuellement présente sur le réactif dans la réalisation de ce test, peut être déterminée selon les méthodes connues avec un substrat approprié permettant par exemple une révélation par colorimétrie, par fluorescence, par luminescence, par potentiométrie, etc...

Avec les anticorps de l'invention, la détection peut être quantitative et permet donc d'effectuer, par les techniques immunologiques, des numérations de levures ou de moisissures dans des produits où elles sont des constituants normaux ou des contaminants.

L'invention concerne également l'utilisation des anticorps anti-levure tels que définis ci-dessus, dans la détection qualitative et/ou quantitative de levures ou de moisissures, en particulier dans des produits alimentaires, qu'il s'agisse de levures ou moisissures normalement présentes ou de levures ou de moisissures susceptibles de contaminer lesdits produits.

L'invention concerne, en particulier, l'application des anticorps polyclonaux et monoclonaux tels que définis ci-dessus, à la détection de levures et/ou de moisissures dans les produits laitiers frais (lait, crème, fromages frais, yaourts, desserts lactés, etc...).

La présente invention a aussi pour objet un procédé de détection et éventuellement de numération de levures et/ou de moisissures présentes normalement ou éventuellement contenues en tant que contaminants dans un produit à tester, caractérisé en ce que lesdits anticorps ou leurs fragments, sont mis en oeuvre, d'une manière connue en soi, dans une méthode immunologique fondée sur la réaction antigène-anticorps, en particulier avec des anticorps marqués.

Outre l'application décrite ci-dessus, les anticorps de l'invention peuvent être utilisés dans un procédé d'élimination des antigènes qu'ils reconnaissent présents dans une substance liquide aqueuse à épurer, ce procédé étant caractérisé par le fait que l'on met en contact ladite substance avec lesdits anticorps fixés sur un support insoluble en phase aqueuse pour réaliser l'immunocaptage, l'immunoabsorption ou l'immunoaffinité desdits antigènes.

En outre, il est clair que les anticorps conformes à la présente invention, de même que les lignées cellulaires dont ils sont issus, s'appliquent à la détection qualitative et/ou quantitative de levures ou de moisissures à des fins d'analyse dans les domaines les plus divers, parmi lesquels on peut citer la biologie clinique, la médecine, la biologie générale, l'agronomie, la zoologie, la physiologie végétale et la mycologie.

Dans le cas de produits pâteux ou gélifiés, la mise en oeuvre du procédé de détection des levures ou moisissures peut éventuellement être précédée d'une mise en condition telle que mise en suspension ou en solution, dans un solvant aqueux, du produit à tester.

Dans le cas où il s'agit de détecter des levures ou moisissures contaminantes, on peut ensuite recueillir lesdites levures ou moisissures éventuellement présentes dans la solution ou suspension examinée, notamment par filtration ou par centrifugation.

L'invention s'étend à des coffrets de détection immunologique qui contiennent, à titre de réactif immunologique, au moins un anticorps, en particulier un anticorps monoclonal, tel que défini précédemment.

Bien entendu, ces coffrets comprennent également les réactifs de mise en évidence de la réaction antigène-anticorps couramment utilisés dans les détections de ce type, à savoir :

a) pour les tests par immunofluorescence, des anticorps couplés à un fluorochrome ainsi que des tampons et des supports appropriés ;

b) pour les tests par voie enzymatique : des anticorps couplés à l'enzyme, les agents révélateurs de l'activité enzymatique, les tampons et les supports appropriés ;

c) pour les tests radioimmunologiques, des anticorps modifiés par un marqueur radioactif, ainsi que les tampons et supports appropriés.

Les exemples suivants illustrent l'invention, sans toutefois la limiter.

EXEMPLE 1 : Préparation d'anticorps polyclonaux chez le lapin.

1. Immunisation

On a injecté à des lapins au temps zéro $6.10^7$ levures tuées, en adjuvant complet de Freund, en injection intradermique multi-points.

Les levures sont tuées par la chaleur (autoclavage à 120°C pendant 20 minutes).

Au temps trois semaines, un rappel est effectué. Pour cela, $6.10^7$ levures sont administrées, avec de l'adjuvant incomplet, en injection sous-cutanée.

Le sang est ensuite prélevé tous les 15 jours et on en sépare le sérum.

Les lapins ont été immunisés avec l'une des levures suivantes :

Candida pseudotropicalis : référence CBS 607
Candida tropicalis : référence CBS 94
Debaryomyces hansenii : référence CBS 770
Candida albicans : référence CBS 562.

## 2. Essai de réponse polyclonale

Les sérums obtenus ont été testés pour déterminer leur activité anti-levure par la méthode immunoenzymatique connue sous le nom de technique ELISA.

Environ $5.10^5$ levures de l'espèce utilisée comme antigène dans le protocole d'immunisation, sont séchées une nuit à 41°C sous un volume de 50µl de solution saline tamponnée au phosphate (P.B.S.) dans les micropuits d'une plaque Dynatech Immulon. Pour éviter une adsorption non spécifique des anticorps, les puits sont saturés à température ambiante pendant une heure avec du P.G.T. (phosphate-gélatine-Tween), c'est-à-dire de la gélatine à 0,25% et du Tween 20 à 0,1% dans du P.B.S.

"Tween 20" est une marque commerciale pour un tensioactif non ionique, le polyoxyéthylène sorbitan monolaurate.

Le sérum à tester est ensuite dilué de puits en puits sous un volume de 50µl. L'incubation est réalisée à température ambiante pendant 1 heure. Après lavage des micropuits avec du P.G.T., 50µl d'une dilution au 1/500 d'immunoglobulines de mouton anti-immunoglobulines de lapin couplées à la peroxydase sont ajoutés. Une série de temoins négatifs a été préparée en incubant le conjugué ci-dessus dans des puits qui ont été incubés avec un sérum pré-immun de lapin, dilué dans les mêmes conditions que le sérum à tester.

Après une heure d'incubation avec le conjugué, les puits sont lavés trois fois avec du P.B.S. et on ajoute ensuite le substrat enzymatique (2,5mM $H_2O_2$ et 2mM de 2,2'-azino-di-(3-éthyl-benzothiazoline-acide sulfonique) dans un tampon acétate-phosphate, pH=6,5). Après 30 minutes, la densité optique de chaque puits est lue dans un lecteur optique Dynatech.

Cette technique ELISA est utilisée pour tester les sérums polyclonaux par réaction homologue et par réactions croisées avec des levures différentes de l'antigène utilisé lors de l'immunisation.

Les résultats obtenus avec les anti-sérums anti-C. pseudotropicalis, anti-C. tropicalis, anti-C. albicans et anti-D. hansenii,sont réunis dans le TABLEAU I qui va suivre.

## TABLEAU I

### Pourcentage de réaction croisée de 4 antisérums de lapins hyperimmuns

| MICRO-ORGANISME | Sous Division ⊕ | ANTI-C. PSEUDO-TROPICALIS | ANTI-C. TROPICALIS | ANTI-C. ALBICANS | ANTI-D. HANSENII |
|---|---|---|---|---|---|
| CANDIDA PSEUDOTROPICALIS | FI | <u>100</u> | 8 | 6 | 15 |
| CANDIDA TROPICALIS | FI | 100 | <u>100</u> | 100 | 100 |
| CANDIDA ALBICANS | FI | 100 | 100 | <u>100</u> | 100 |
| DEBARYOMYCES HANSENII | A | 100 | 66 | 73 | <u>100</u> |
| TRICHOSPORON CUTANEUM | FI | 85 | 16 | 21 | 23 |
| TORULOPSIS GLABRATA | FI | 26 | 43 | 64 | 38 |
| CRYPTOCOCCUS NEOFORMANS | FI | 15 | 4 | 0 | 5 |
| SACCHAROMYCES CARLSBER-GENSIS | A | 100 | 16 | 7 | 15 |
| SACCHAROMYCES CEREVISIAE | A | 20 | 5 | 3 | 21 |
| SACCHAROMYCES CEREVISIAE MUTEE | A | 85 | 14 | 6 | 15 |
| STREPTOCOCCUS THERMOPHI-LUS | | 13* | 0 | 0 | 0 |
| LACTOBACILLUS BULGARICUS | | 13* | 0 | 0 | 0 |

\* pourcentage déterminé sur les deux souches en mélange.

⊕ FI = fungi imperfecti ;

   A = ascomycetes.

Le pourcentage de réactivité croisée est calculé par rapport à la levure utilisée pour l'immunisation (à laquelle on attribue la valeur 100).

Les microorganismes testés sont les suivants :

D. hansenii (souche CBS 770) ;

C. tropicalis (souche CBS 94) ;

Ferment lactique (CO 32) : mélange de Streptococcus thermophilus et de Lactobacillus bulgaricus (fourni par BSN) ;

C. pseudotropicalis (souche CBS 607) ;

C. albicans (souche CBS 562) ;

S. cerevisiae mutée au niveau de sa paroi (souche LB6-50) ;

T. glabrata (souche CBS 138) ;

Tr. cutaneum (souche CBS 2466) ;

Cr. neoformans (souche CBS 464) ;

S. cerevisiae (souche CBS 1171) ;

S. carlsbergensis (souche CBS 7003).

La référence CBS signifie CENTRAALBUREAU VOOR SCHIMMELCULTURES, BAARN (PAYS-BAS).

La souche LB6-50 est une souche de S. cerevisiae mutée au niveau de sa paroi (fournie par University of California, Biochemistry Department, BERKELEY, Cal.94720 C.E. BALLOU Bc 50).

3.Détection de levures par la technique ELISA

Des tests de détection des levures ont été réalisés afin de déterminer la sensibilité de la technique ELISA lors de l'utilisation de sérums polyclonaux, en utilisant du sérum de lapin anti-C. tropicalis. Le protocole décrit précédemment est légèrement modifié : les levures homologues sont diluées de puits en puits, puis séchées dans les plaques noires Dynatech. Les micropuits sont saturés avec le tampon P.G.T. durant 1 heure, puis on ajoute 50$\mu$l d'une dilution fixe de sérum de lapin anti-levure.

Après une heure, l'anticorps de mouton anti-immunoglobulines de lapin, couplé à la phosphatase alcaline, est ajouté. L'incubation est réalisée à température ambiante.

Après trois lavages en P.B.S., le substrat enzymatique est ajouté (diéthanolamine 1M, $MgCl_2$ 5mM, 4 méthylumbelliferylphosphate 0,1mM, pH 9,8). La lecture est effec tuée sur un lecteur Microfluor Dynatech.

La sensibilité de la détection des levures est augmentée par purification des immunoglobulines par chromatographie sur colonne échangeuse d'ions : DEAE TRISACRYL.

Les IgG de lapin sont éluées avec un tampon Nacl 35mM ; Tris 25mM pH 8,8.

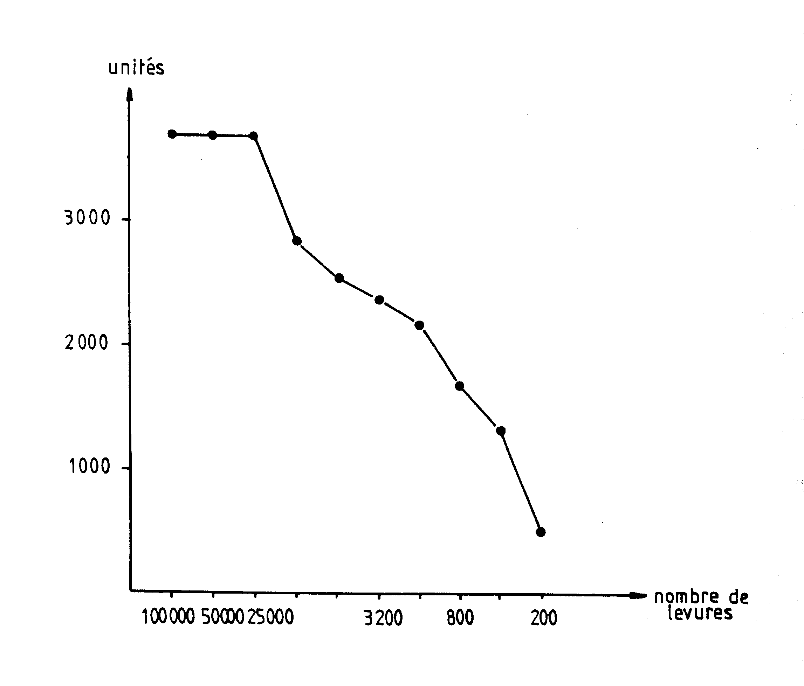

La courbe de quantification de levures C. tropicalis par la fraction anticorps d'un sérum de lapin anti-C. tropicalis, est représentée à la figure unique annexée, avec en abscisse le nombre de levures et en ordonnée la densité optique ; dans le cas représenté le seuil minimal de détection s'est avéré être de 200 levures environ.

EXEMPLE 2 : Préparation d'anticorps monoclonaux.

1. Immunisation

On a immunisé des souris femelles Balb/c en leur injectant une fois par semaine pendant trois semaines consécutives, par voie intra-veineuse, $10^7$ levures, tuées par la chaleur par autoclavage à 120°C pendant 20 minutes. Après une période de repos de trois mois, les souris ont eu un rappel par voie intra-veineuse avec la même quantité de levures trois jours avant le prélèvement des splénocytes pour la fusion.

La levure utilisée pour l'immunisation a été : Debariomyces hansenii.

2. Fusion

Des cellules de myélome de souris PIE sont utilisées pour la fusion. Elles ont été sélectionnées pour leur sensibilité à l'aminoptérine et cultivées sur le milieu RPMI contenant 10% de sérum de veau foetal, 2mM de glutamine, 1mM de pyruvate de sodium, 100 UI/ml de pénicilline et 100$\mu$g/ml de streptomycine.

Les splénocytes ont été dispersés par injection du milieu RPMI exempt de sérum dans la rate des souris hyperimmunisées et lavés trois fois dans du milieu RPMI avant fusion.

Les cellules de myélome et les splénocytes ont été ensuite fusionnés dans un rapport de une cellule de myélome pour 10 splénocytes, en présence de 41% de polyéthylèneglycol d'un poids de 1500 (Merck) selon la méthode de KOHLER et MILSTEIN (Nature, 256, 495-497, 1975), puis lavées dans le milieu RPMI et remises en suspension dans 100ml du milieu RPMI complet. Les cellules ont ensuite été redistribuées dans des microplaques Nunclon (24 puits par plaque) à raison de 1ml/puits contenant 2,2.$10^5$ cellules. Après 24 heures, on a ajouté 1ml/puits de milieu constitué du milieu RPMI complet contenant 0,1mM d'hypoxanthine, 0,4$\mu$M d'aminoptérine et 16$\mu$m de thymidine. Des parties aliquotes de milieu sélectif ont été remplacées les 3ème, 6ème et 10ème jours après la fusion. Après 15 jours, les hybridomes survivants ont été cultivés sur le milieu RPMI complet, complété avec de l'hypoxanthine et de la thymidine et, 15 jours après la fusion, on a recherché la présence d'anticorps anti-levures dans les surnageants de culture. Les clones ont été ensuite cultivés progressivement sur le milieu RPMI complet normal.

Les cultures positives ont été clonées par la méthode des dilutions limites selon OI V.T. et HERZENBERG, L.A. 1980 Immunoglobulin-producing hybrid cell lines, in "Selected Methods in Cellular Immunology" (Eds. Mishell, R.B. and Shrigi, S.M., p.351, Fillman, San Franciso).

Les cellules ont été redistribuées dans les puits d'une microplaque Nunclon, à raison de 0,5 cellule en moyenne par puits, avec 3.10$^5$ thymocytes à titre de cellules nutritives. Après 10 jours, les puits contenant des clones uniques ont été sélectionnés et 5 jours plus tard, les surnageants ont été testés pour déterminer la présence d'anticorps anti-levures.

Trois clones issus d'une culture primaire positive, ont été sélectionnés et injectés à des souris Balb/c pour obtenir de grandes quantités d'anticorps. A cet effet, des souris femelles agées Balb/c ont été stimulées par une injection intrapéritonéale de 0,3 ml de tétraméthyl-pentadécane. Après 4 jours, 20 millions de cellules hybrides ont été injectées aux souris. Au bout de 15 jours, les fluides d'ascites ont été récoltés et leur activité anti-levure et anti-moisissure a été testée.

Trois clones de cellules hybrides obtenus ont été déposés auprès de la Collection Nationale de Culture de Microorganismes de l'Institut Pasteur, Paris (France) sous le numéro I-501 le 6 Décembre 1985 (IG$_7$), I-500 le 6 Décembre 1985 (DG$_3$) et I-509 le 22 Janvier 1986 (IE$_3$).

### 3. Caractérisation des anticorps monoclonaux par la méthode ELISA

Les anticorps obtenus selon l'exemple 2 ont été testés pour déterminer leur activité anti-D. hansenii par la méthode immunoenzymatique connue sous le nom de méthode ELISA, décrite précédemment.

Les micropuits d'une plaque Immulon Dynatech ont été incubés avec 5.10$^5$ levures D. hansenii et séchés une nuit. Les puits sont ensuite saturés avec le tampon P.G.T. puis incubés avec 50µl de surnageant de culture d'hybridome ou de fluide ascitique durant 1 heure à température ambiante. Les puits sont alors lavés et on ajoute 50µl d'immunoglobulines de lapin anti-immunoglobuline de souris marquée à la peroxydase, diluées au 1/500. Après 1 heure d'incubation, trois lavages au P.B.S. sont effectués et le substrat est ajouté.

Afin de sélectionner les anticorps permettant de rechercher la présence de levures et/ou de moisissures dans le yaourt, les surnageants de culture d'hybridome sont également testés sur Streptococcus thermophilus et Lactobacillus bulgaricus. Dans ce cas, le test ELISA doit être négatif, afin d'éliminer tout anticorps susceptible de présenter une quelconque réaction croisée ultérieure lors de la recherche de levures ou de moisissures contaminantes dans le yaourt.

Les clones retenus doivent donc reconnaître D. hansenii et ne pas présenter de réaction croisée avec les ferments lactiques.

Les hybridomes BC$_4$, IG$_7$, DG$_3$ et IE$_3$ présentent ces deux caractéristiques simultanément.

Le clonage, réalisé comme décrit précédemment, a permis d'obtenir 35 clones dont trois ont été propagés en ascites (désignés par DG$_3$, IE$_3$, IG$_7$).

### 4. Caractérisation des anticorps monoclonaux obtenus à partir des fluides ascitiques.

Les isotypes des anticorps monoclonaux obtenus dans les fluides ascitiques ont été déterminés par la méthode ELISA indirecte en utilisant des anticorps polyclonaux monospécifiques de lapin contre les différents isotypes d'immunoglobulines murines.

Les fluides ascitiques obtenus après injection d'un clone contiennent des anticorps anti-D. hansenii.

Les anticorps monoclonaux anti-D. hansenii ont ensuite été testés par la méthode ELISA afin de déterminer s'il existe ou non des réactions croisées envers des levures ou des moisissures différentes.

Dans cette expérience, on utilise 500.000 levures, ou 500.000 spores, ou 10µg de mycelium par puits. Le fluide ascitique est dilué au 1/2000 ou au 1/10.000. Les résultats obtenus montrent que les anticorps monoclonaux reconnaissent notamment D. hansenii, C. pseudotropicalis, C. albicans, C. tropicalis, T. glabrata, Trichosporon cutaneum, Saccharomyces carlsbergensis, avec des intensités comparables.

D'autres levures tells que S. cerevisiae et Cryptococcus neoformans, sont moins bien reconnues.

Ces résultats sont représentés dans le Tableau II ci-après.

9

## TABLEAU II

Pourcentage de réaction croisée des surnageants
de culture (BC4) et d'anticorps monoclonaux
($IG_7$,$DG_3$,$IE_3$) anti-D. hansenii envers différentes levures
et ferments lactiques

| MICRO-ORGANISME | Sous Division ⊕ | BC4 | IG7 | DG3 | IE3 |
|---|---|---|---|---|---|
| DEBARYOMYCES HANSENII | A | 100 | 100 | 100 | 100 |
| CANDIDA TROPICALIS | FI | 84 | 80 | 90 | 64 |
| CANDIDA PSEUDOTROPICA-LIS | FI | 77 | 105 | 75 | 47 |
| CANDIDA ALBICANS | FI | 95 | 140 | 80 | 87 |
| TORULOPSIS GLABRATA | FI | 92 | 95 | 86 | 45 |
| TRICHOSPORON CUTANEUM | FI | 120 | 100 | 77 | 91 |
| CRYPTOCOCCUS NEOFORMANS | FI | 18 | 13 | 0 | 0 |
| SACCHAROMYCES CARLS-BERGENSIS | A | 82 | 100 | 95 | 100 |
| SACCHAROMYCES CERE-VISIAE | A | 32 | 32 | 18 | 50 |
| SACCHAROMYCES CERE-VISIAE MUTEE | A | 100 | 120 | 121 | 130 |
| CANDIDA UTILIS | FI | 100 | 100+ | 90 | 90 |
| CANDIDA LIPOLYTICA | FI | | 100+ | | 100+ |
| CANDIDA PARAPSILOSIS | FI | | 100+ | | 100+ |
| CANDIDA KRUSEI | FI | | 100+ | | 100+ |
| CANDIDA GUILLERMONDII | FI | | 100+ | | 100+ |
| CANDIDA DIDDENSII | FI | | 100+ | | 100+ |
| CANDIDA ALBICANS | FI | | 100+ | 80 | 90 |
| RHODOTORULA RUBRA | FI | | 80 | | 50 |
| PICHIA MEMBRANEFACIENS | A | | 100+ | | 100+ |
| HANSENULA SATURNUS | A | 100+ | 80 | 100+ | 80 |
| HANSENULA ANOMALA | A | | 70 | | 80 |
| KLUYVEROMYCES LACTIS | A | | 100+ | 100+ | 100 |
| KLUYVEROMYCES FRAGILIS | A | | 100+ | | 100+ |
| KLUYVEROMYCES MARXIANUS | A | | 100+ | | 100 |
| SACCHAROMYCES POMBE | A | 100+ | 100+ | 100+ | 90 |
| SACCHAROMYCES ROUXII | A | | 80 | | 100 |
| SACCHAROMYCES BAILLII | A | | 100+ | | 100+ |
| SACCHAROMYCES FLORENTINIUM | A | | 100+ | 90 | 70 |
| ZYGOSACCHAROMYCES BAILLII | A | | 90 | | 80 |
| ZYGOSACCHAROMYCES BISPORUS | A | | 50 | | 50 |

\* FI = fungi imperfecti ;
A = ascomycetes.

Les résultats obtenus montrent en outre que les surnageants de culture BC4 reconnaissent entre autres le mycelium et les spores de M. racemosus, E. repens, P. verrucosum, P. frequentans, P. camembertii, P.

# 0 237 384

roquefortii, A. versicolor, A. fumigatus, A. tenissima, U. cartarum, T. longibrachatium, P. malorum, G. candidum, avec des intensités comparables. Les anticorps monoclonaux (IG7, DG3, IE3) reconnaissent certaines moisissures avec des intensités comparables et certaines moisissures avec des intensités différentes. Ces résultats sont représentés dans le tableau III ci-après.

## TABLEAU III

Pourcentage de réaction croisée des surnageants de culture (BC4) et d'anticorps monoclonaux (IG7, DG3, IE3) anti-D. hansenii envers différentes moisissures.

| MICRO-ORGANISME | Sous-division* | BC4 | IG7 | DG3 | IE3 |
|---|---|---|---|---|---|
| Mucor racemosus spore | Z | 100 + | 0 | 8 | 43 |
| Mucor racemosus myc | Z | 100 + | 0 | 10 | 10 |
| Mucor plumbeus spore | Z | 57 | 0 | 9 | 10 |
| Mucor plumbeus myc | Z | 100 + | 0 | 11 | 8 |
| Eurotium repens spore | A | 100 + | 32 | 45 | 45 |
| Eurotium repens myc | A | 100 + | 21 | 35 | 23 |
| Penicillium verrucosum spore | FI | 45 | 12 | 33 | 30 |
| Penicillium verrucosum myc | FI | 100 + | 0 | 23 | 30 |
| Penicillium frequentans spore | FI | 80 | 0 | 4 | 30 |
| Penicillium frequentans myc | FI | 100 + | 0 | 41 | 59 |
| Penicillium camemberti spore | FI | 100 + | 38 | 40 | 42 |
| Penicillium camemberti myc | FI | 100 + | nd | 48 | 30 |
| Penicillium roqueforti spore | FI | 100 + | 0 | 9 | 10 |
| Penicillium roqueforti myc | FI | 100 + | 43 | 59 | 44 |
| Aspergillus versicolor spore | FI | 100 + | 12 | 13 | 35 |
| Aspergillus versicolor myc | FI | 100 + | 0 | 81 | 73 |
| Aspergillus fumigatus spore | FI | 89 | 0 | 13 | 12 |
| Aspergillus fumigatus myc | FI | 100 | nd | 20 | 28 |
| Alternaria tenissima spore | FI | nd | nd | nd | nd |
| Alternaria tenissima myc | FI | 100 + | nd | nd | nd |
| Ulocladium cartarum spore | FI | nd | nd | nd | nd |
| Ulocladium cartarum myc | FI | 100 + | nd | 100 + | 100 + |
| Trichoderma longibrachatium spore | FI | 100 + | 0 | 41 | 44 |
| Trichoderma longibrachatium myc | FI | 100 + | 87 | 55 | 70 |
| Phialophora malorum spore | FI | 100 + | 43 | 44 | 51 |
| Phialophora malorum myc | FI | 100 + | nd | 100 + | 94 |
| Fusarium spore | FI | 100 + | 58 | 97 | 100 |
| Fusarium myc | FI | 100 + | nd | 76 | 70 |
| Geotrichum candidum spore | FI | 100 + | 75 | 97 | 100 |
| Geotrichum candidum myc | FI | 100 + | 75 | 76 | 70 |

* Z = zygomycotina ;

A = ascomycotina ,

FI = fungi imperfectii (deuteromycotina).

11

La majorité des ferments lactiques, notamment ceux utilisés en France dans la préparation industrielle des yaourts, n'est pas reconnue.

Les résultats sont résumés dans le tableau IV ci-après :

### TABLEAU IV

Pourcentage de reaction croisée des surnageants de culture (BC4) et d'anticorps monoclonaux (IG7,DG3,IE3) anti-D. hansenii envers differentes bacteries lactiques.

| MICRO-ORGANISME | GROUPE | BC4 | IG7 | DG3 | IE3 |
|---|---|---|---|---|---|
| STREPTOCOCCUS THERMO-PHILUS | L* | 0 | 0 | 0 | 0 |
| STREPTOCOCCUS LACTIS | L | 25 | 20 | 30 | 20 |
| STREPTOCOCCUS DIACE-TYLACTIS | L | 15 | 15 | 15 | 15 |
| LACTOBACILLUS BULGA-RICUS | L* | 0 | 0 | 0 | 0 |
| LACTOBACILLUS ACIDO-PHILUS | L | 0 | 0 | 0 | 0 |
| LACTOBACILLUS BREVIS | L | 0 | 0 | 0 | 0 |
| LACTOBACILLUS HELVETICUS | L | 0 | 0 | 0 | 0 |
| LACTOBACILLUS HELVETICUS VAR. JUGURTI | L | 0 | 0 | 0 | 0 |
| LACTOBACILLUS LACTIS | L | 0 | 0 | 0 | 0 |
| LACTOBACILLUS CASEI RHAMNOSUS | L | 11 | 21 | 16 | 16 |
| LACTOBACILLUS CASEI CASEI | L | 27 | 33 | 40 | 23 |
| BIFIDOBACTERIUM BIFIDUM | L | 0 | 13 | 14 | 0 |

L = bacteries lactiques

L* = bacteries lactiques communément utilisées en fermentation yaourtiére.

Les microorganismes testés sont les mêmes que dans l'exemple 1, sauf en ce qui concerne les ferments lactiques qui, outre ceux mentionnés à l'exemple 1, sont les suivants :
Lactobacillus acidophilus BSN E027
Lactobacillus brevis BSN H001
Lactobacillus helveticus BSN D013
Lactobacillus helveticus var. jugurti BSN D008
Lactobacillus lactis BSN B043
Lactobacillus casei rhamnosus BSN F005
Lactobacillus casei casei BSN F001
Bifidobacterium bifidum BSN I010
Streptococcus thermophilus BSN A107
Streptococcus lactis BSN L032

Streptococcus diacétylactis BSN M01

Les moisissures testées étaient les suivantes :

Mucor racemosus MHN

Mucor plumbeus MHN

Eurotium repens MHN

Penicillium verrucosum BSN S002

Penicillium frequentans BSN S004

Penicillium roquefortii MHN

Penicillium camembertii MHN

Aspergillus versicolor BSN S003

Aspergillus fumigatus BSN S007

Geotrichum candidum ULB

Alternaria tenissima BSN S005

Ulocladium cartorum BSN S006

Trichoderma longibrachatium BSN S008

Phialophora malorum BSN S015

Fusarium BSN S018

MHN = Museum d'Histoire Naturelle de Paris (Laboratoire de Cryptogamie)

ULB = Université Libre de Bruxelles (Laboratoire de Microbiologie et d'immunologie).

EXEMPLE 3 : Recherche de levures dans le lait

La technique de détection des levures dans le lait se fait par la méthode ELISA.

Le lait contaminé est dilué dans une solution saline (P.B.S.), est réparti sous 50µl dans les puits d'une plaque Dynatechn, puis séché pendant une nuit à 41°C.

Un témoin négatif de lait non contaminé est traité dans les mêmes conditions.

Les puits sont saturés pendant une heure avec du P.G.T.

Un sérum polyclonal anti-levure de lapin est ajouté sous 50µl et au au 1/400e dans les puits. Il est incubé pendant une heure à température ambiante.

Après lavage des micropuits avec du P.G.T., 50µl d'une dilution au 1/500e d'immunoglobulines de mouton anti-immunoglobulines de lapin couplées avec de la peroxydase sont ajoutés.

Après une heure d'incubation avec le conjugué, les puits sont lavés avec du P.B.S., et on ajoute le substrat enzymatique ABTS (2,2'-azino-di-3-éthyl-benzothiazoline-acide sulfonique).

Après 30 minutes, la densité optique de chaque puits est lue dans un lecteur optique Dynatech.

EXEMPLE 4 : Recherche de moisissures dans la crème fraîche

La technique de détection des moisissures dans la crème fraîche se fait par la méthode ELISA.

La crème fraîche (100 microlitres) est solubilisée, puis est filtrée sur des filtres 5,0µm d'une plaque MILLITITER (Millipore). A la crème fraîche solubilisée (200µl) obtenue, on ajoute 100 nanogrammes de mycelium de Penicillium verrucosum. Un témoin négatif de crème fraîche non contaminée est traité dans les mêmes conditions.

Les filtres sont saturés pendant une heure avec du PGT. L'anticorps monoclonal (1 microlitre de l'ascite IE3, complété à 1 millilitre) est ajouté par fractions de 50µl sur les filtres et est incubé pendant une heure à température ambiante.

Après plusieurs lavages des microfiltres avec du PGT, 50µl d'une solution au 1/500e d'immunoglobuline de lapin (origine KPL) anti-immunoglobuline de souris couplé à la peroxydase sont ajoutés.

Après une heure d'incubation avec le conjugué, les filtres sont lavés avec du PBS, et on ajoute le substrat enzymatique, le dichlorhydrate de 0-dianisidine (3,3'-diméthoxybenzidine) (15mg) + 5ml méthanol + 25 ml PBS + 1µl $H_2O_2$ (à 30%).

Après 15 minutes, l'apparition d'une coloration rouge-brun note la présence de moisissures.

**Revendications**

1. Anticorps polyspécifiques, caractérisés par le fait qu'ils sont capables de reconnaître au moins une levure et au moins une moisissure.

2. Anticorps selon la revendication 1, caractérisés par le fait qu'ils sont capables de reconnaître une première levure et au moins une autre levure différente de la première par son genre et/ou son espèce.

3. Anticorps polyspécifiques selon la revendication 1 ou 2, caractérisés par le fait qu'ils sont capables de reconnaître au moins une première et une deuxième levures prises dans le groupe qui comprend notamment les levures des genres Candida, Saccharomyces, Trichosporon, Debaryomyces, Torulopsis, Cryptococcus, Rhodotorula, Pichia, Hansenula, Kluyveromyces et Zygosaccharomyces.

4. Anticorps selon l'une quelconque des revendications 1 à 3, caractérisés par le fait qu'il sont capables de reconnaître au moins un moisissure choisie parmi celles des genres Penicillium, Aspergillus, Mucor, Geotrichum, Alternaria, Ulocladium, Trichoderma, Phialophora, Fusarium et Eurotium.

5. Anticorps selon l'une des revendications précédentes, caractérisés par le fait qu'il s'agit d'anticorps polyclonaux.

6. Anticorps selon l'une quelconque des revendications 1 à 4, caractérisés par le fait qu'il s'agit d'anticorps monoclonaux.

7. Anticorps selon la revendication 6, caractérisés par le fait qu'ils sont sécrétés par une lignée cellulaire de cellules hybrides choisie parmi les celles déposées sous les numéros I-500, I-501 et I-509 à la Collection Nationale de Microorganismes de l'Institut Pasteur.

8. Anticorps selon l'une quelconque des revendications précédentes, caractérisés par le fait qu'ils sont fragmentés par voie enzymatique ou chimique.

9. Anticorps selon l'une quelconque des revendications 1 à 8, caractérisés par le fait qu'ils sont modifiés par marquage et/ou fixés sur un support solide.

10. Anticorps selon l'une quelconque des revendications précédentes, caractérisés par le fait qu'ils ne donnent pas de réactivité croisée, ou ne donnent qu'une faible réactivité croisée avec les ferments lactiques.

11. Lignées cellulaires hybrides, caractérisés par le fait qu'elles sécrètent des anticorps tels que définis dans la revendication 6.

12. Lignées cellulaires selon la revendication 11, caractérisées par le fait qu'elles résultent de la culture et du clonage de cellules hybrides choisies parmi celles déposées en date du 6 Décembre 1985 sous les numéros I-500, I-501 et en date du 22 Janvier 1986 I-509, auprès de la Collection Nationale de Culture des Microorganismes de l'Institut Pasteur.

13. Procédé de préparation d'anticorps polyspécifiques tels que définis dans l'une quelconque des revendications 1 à 10, caractérisé par le fait qu'il comprend une première étape, dans laquelle on immunise des animaux vertébrés, selon les techniques connues, et une seconde étape dans laquelle on recueille les anticorps des animaux immunisés.

14. Procédé selon la revendication 13, caractérisé par le fait que l'on effectue l'immunisation à l'aide de cellules de levure.

15. Procédé selon l'une quelconque des revendications 13 et 14, caractérisé par le fait que la seconde étape comprend un premier stade qui consiste à fusionner des cellules lymphoïdes des animaux immunisés avec des cellules de vertébrés cultivables in vitro, et un deuxième stade qui consiste à cloner les cellules hybrides obtenues.

16. Procédé de détection et éventuellement de numération de levures et/ou de moisissures présentes normalement ou éventuellement contenues en tant que contaminants dans un produit à tester, caractérisé par le fait que lesdits anticorps, tels que définis dans l'une quelconque des revendications 1 à 10, ou leurs fragments, sont mis en oeuvre d'une manière connue en soi, dans une méthode immunologique fondée sur la réaction antigène-anticorps.

17. Application du procédé selon la revendication 16 à la détection qualitative et/ou quantitative de levures et/ou de moisissures dans des produits alimentaires et/ou des substances biologiques.

18. Application selon la revendication 17, à la détection qualitative et/ou quantitative de levures et/ou de moisissures dans des produits lactés contenant des ferments lactiques avec lesquels les anticorps mis en oeuvre ne donnent pas de réactions croisées.

19. Coffret ou trousse de détection par voie immunologique de levures dans des substances biologiques ou alimentaires, caractérisé par le fait qu'il contient outre des tampons et des supports appropriés, au moins un anticorps polyspécifique selon l'une quelconque des revendications 1 à 10, et des agents de mise en évidence de la réaction antigène-anticorps.

Office européen
des brevets

**RAPPORT DE RECHERCHE EUROPEENNE**

Numéro de la demande

EP 87 40 0282

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | INFECTION AND IMMUNITY, vol. 48, no. 3, juin 1985, pages 806-812, American Society for Microbiology; B.V. KUMAR et al.: "Cross-reacting human and rabbit antibodies to antigens of histoplasma capsulatum, candida albicans, and saccharomyces cerevisiae" * En entier * | 1-5,8, 9,13, 14,16 | C 07 K 15/00<br>C 12 P 21/00<br>C 12 N 5/00<br>C 12 N 15/00<br>G 01 N 33/569<br>G 01 N 33/577//<br>(C 12 P 21/00<br>C 12 R 1:91 ) |
| A | B.D. DAVIS et al.: "Microbiology", 3ème édition, 1980, pages 836-837, Harper International Edition * Page 836, colonne de droite, ligne 28 - page 837, colonne de gauche, ligne 25 * | 1-19 | |
| A | J. LODDER: "THE YEASTS", 2ème édition revue et élargie, 1974, page 3, North-Holland Publishing Co., Amercian Elsevier Publishing Co., New York, US * Page 3, lignes 11-16 * | 1-19 | **DOMAINES TECHNIQUES RECHERCHES (Int. Cl.4)**<br><br>C 12 P<br>G 01 N<br>A 61 K |
| A | CHEMICAL ABSTRACTS, vol. 93, no. 13, 29 septembre 1980, page 510, résûmé no. 130551m, Columbus, Ohio, US; & JP-A-80 68 299 (SAPPORO BREWERIES, Ltd) 22-05-1980 * Résumé * | 1-3,13 ,14,16 ,17 | |

--- -/-

Le présent rapport de recherche a été établi pour toutes les revendications

| Lieu de la recherche | Date d'achèvement de la recherche | Examinateur |
|---|---|---|
| LA HAYE | 29-05-1987 | CHARLES D.J.P.I.G. |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 543 969 (KUREHA KAGAKU KOGYO KABUSHIKI KAISHA) <br> * Page 1, lignes 1-19; page 3, lignes 1-8, ligne 31 - page 4, ligne 12; page 4, ligne 21 - page 5, ligne 3; page 12, lignes 12-32; revendications 1-42 * <br><br> --- | 1-3,6, 13-19 | |
| A | INFECTION AND IMMUNITY, vol. 43, no. 3, mars 1984, pages 966-972, American Society for Microbiology; D.L. BRAWNER et al.: "Variability in expression of a cell surface determinant on candida albicans as evidenced by an agglutinating monoclonal antibody" <br> * En entier * <br><br> --- | 1-3,6, 13-16 | |
| A | CHEMICAL ABSTRACTS, vol. 105, no. 23, 8 décembre 1986, page 283, résumé no. 20541w, Columbus, Ohio, US; M. VAI et al.: "Immunological cross-reactivity of fungal and yeast plasma membrane proton-ATPase" <br> * Résumé * <br><br> ----- | 1,3,5 | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 29-05-1987 | CHARLES D.J.P.I.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82